# EUROPÄISCHE PATENTSCHRIFT

(11) **EP 1 820 782 B1**
(45) Veröffentlichungstag und Bekanntmachung des Hinweises auf die Patenterteilung: **30.12.2009**
(21) Anmeldenummer: 07001958.3
(22) Anmeldetag: 30.01.2007
(51) Int. Cl.: C02F 3/28, C02F 11/04

(54) **Verfahren zur Behandlung von Schlämmen**
Process for the treatment of sludges
Procédé pour le traitement des boues

(30) Priorität: 08.02.2006 DE 102006006166
(43) Veröffentlichungstag der Anmeldung: 22.08.2007
(73) Patentinhaber: Holm, Niels Christian, 32425 Minden (DE)
(72) Erfinder: Holm, Niels Christian, 32425 Minden (DE)
(74) Vertreter: Biehl, Christian

(56) Entgegenhaltungen:
- EP-A1- 0 737 651
- US-A- 4 632 758
- US-A- 5 630 942
- US-A1- 2003 075 501

## Beschreibung

Die Erfindung betrifft ein Verfahren zur anaeroben Behandlung von Abwassern und Schlämmen in einem Faulbehälter.

Aus der DE 10 2004 003 071 A1 ist ein Verfahren zur Abwasserreinigung bekannt, bei dem das anfallende Permeat zum Waschen des ausgefaulten Schlamms rückgeführt wird. Aus der DE 40 42 223 A1 ist es bekannt, in einem Abwasseraufbereitungsrecktor anfallende Sinkstoffe im Kreis zu führen und in übergeordneten Prozessen weiter zu verwenden. Ein ähnliches Verfahren ist aus der US-A-4 632 758 bekannt.

Die US-A-5 630 942 beschreibt ein aerobes Verfahren, bei dem Schlamm diskontinuierlich aus einem Faulreaktor abgezogen wird und über einen Speicher rezykliert werden kann.

Für die Behandlung von Schlämmen (z.B. Primär- oder Überschussschlämmen aus Kläranlagen), Gülle von Rindern, Schweinen oder Hühnern oder auch sogenannte Nawaros (nachwachsende Rohstoffe) werden die eingangs genannten Anlagen üblicherweise für eine Aufenthaltszeit ausgelegt, die bei mesophilen Anlagen ca. 20 - 30 Tage und bei thermophilen Anlagen ca. 10 - 15 Tagen beträgt. Bei solchen Anlagen ist die Aufenthaltszeit der partikulären Bestandteile der Faulschlämme (das als anaerobes Schlammalter bezeichnet werden kann) in dem Behälter (die die anaeroben Prozessumwandlungen bewerkstelligen und eine prozessstabile und weitgehende anaerobe Ausfaulung der Substrate gewährleistet) nahezu identisch mit der Aufenthaltszeit der hydraulischen Bestandteile. Insbesondere gewährleisten hohe Schlammalter die stabile Etablierung methanogener Bakterienpopulationen, die für den letzten und entscheidenden Prozessschritt der Ausfaulung, die Methangärung, verantwortlich sind.

Ein solcher Anlagenbetrieb, bei der die Aufenthaltszeit der partikulären Bestandteile nahezu identitisch ist mit der Aufenthaltszeit der hydraulischen Bestandteile (bei der also keine Entkoppelung der Aufenthaltszeit der rein löslichen Phase von der der partikulären Phase gegeben ist) findet üblicherweise statt, wenn das zu behandelnde Substrat hoch konzentriert ist. Das ist z.B. gegeben, wenn Klärschlämme mit einer TS-Konzentration (TS = Trockensubstanz) >= 5 % TS (= 50 g/l TS) verwendet werden oder wenn flüssiges Abwasser einen organischen Kohlenstoffanteil (CSB = chemischer Sauerstoff Bedarf) ausweist, aus der eine TS-Konzentration nach der Ausfaulung von möglichst > 3 % TS resultiert.

Je geringer bei dieser Art der Verfahrenstechnik mit volldurchmischten Reaktoren ohne Hydraulikentkoppelung die Substratkonzentration wird, umso ungünstiger ist die Wirtschaftlichkeit hinsichtlich Gasausbeute/m³ Reaktorvolumen.

Daher werden Klärschlämme mit einer TS - Konzentration deutlich unter 5 % TS oft einer maschinelle Voreindickung genannten Aufkonzentrierung auf 5 - 6 % TS unterzogen.

Flüssiges Abwasser mit einer geringen CSB Konzentration wird dagegen oft mittels spezieller anaerober Verfahrenstechniken behandelt, die eine Hydraulikentkoppelung gewährleisten. Hierzu gehört das weit verbreitete, beispielsweise in der DE 40 42 223 beschriebene UASB Verfahren. Bei diesem Verfahren wird das auszufaulende, flüssige Substrat einem UASB Reaktor im Sohlbereich zugeführt. Der untere Reaktorbereich ist mit anaeroben Granulas (das ist die Anaerob-Biologie) gefüllt. Beim Durchströmen dieses Bereiches wird das Abwasser ausgefault und nur die überwiegend flüssige ausgefaulte Phase tritt über den oberen Reaktorbereich in den Auslauf ein. Damit wird die Faulschlammbiomasse von der reinen Hydraulik entkoppelt und die Aufenthaltszeit dieser Biomasse gegenüber der reinen Aufenthaltszeit der hydraulischen Bestandteile stark erhöht. Das Volumen des UASB Reaktors kann somit kleiner ausgelegt werden als nach üblichen rein hydraulischen Erfordernissen bei volldurchmischten Reaktoren.

Auch andere Verfahren zur Faulschlammanreicherung wie Festbettverfahren (US 2003/075501 A1, US A 4 632 758 und US A 5 630 942 erfordern Einbauten im eigentlichen Faulreaktor.

Der Erfindung liegt damit die Aufgabe zugrunde, ein Verfahren und eine Anlage zur Durchführung dieses Verfahrens zu schaffen, die ohne aufwendige Einbauten im Faulreaktor einen wirtschaftlichen Betrieb bei großer Aufenthaltszeit der partikulären Bestandteile ermöglichen.

Erfindungsgemäß wird diese Aufgabe durch die Merkmale des Anspruchs 1 gelöst, der Unteranspruch gibt eine vorteilhafte Ausgestaltung der Erfindung an.

Erfindungsgemäß wird also ein Verfahren vorgeschlagen, nach dem diskontinuierlich aus dem eigentlichen Anaerob-Reaktor ein Teilstrom (3 - 20 % des Reaktorvolumens) entnommen und in einen sogenannten Faulschlammanreicherungsreaktor FAR geführt wird. Dieser verfügt, bezogen auf die Flüssigphase, über eine direkte Verbindung zum eigentlichen Anaerob-Reaktor, die automatisch verschlossen/geöffnet werden kann, so dass im geöffneten Zustand kommunizierende Wassersäulen vorliegen.

Nach einer bevorzugten Ausführungsform dieser Erfindung fördert eine Gaspumpe das Gas aus dem FAR in einen externen Gasspeicher. Möglich ist auch die Förderung des Gases aus dem FAR 2 in den Anaerob-Reaktor 1.

Nach einer weiteren bevorzugten Ausführungsform wird das Faulschlammkonzentrat nur dem eigentlichen Anaerob-Reaktor entnommen, z.B. über eine Ableitung 9. Möglich ist auch eine Entnahme aus dem FAR z.B. über eine Ableitung 10 oder der es wird nur über die Klarwasserfraktion über die Ableitung 8 entnommen, dann muss nachfolgend allerdings noch eine Schlamm/Wasser Trennung stattfinden.

Nach einer weiteren bevorzugten Ausführungsform wird das Klarwasser, nachdem die Faulschlammkonzentratrückführung in den Anaerob-Reaktor erfolgt ist, entnommen. Möglich ist auch die Klarwasserentnahme direkt vor der Faulschlammkonzentratrückführung. Dann wird mit Beginn des nächsten Zyklusses allerdings eine vergleichsweise hohe Faulschlammkonzentration im FAR vorliegen.

Nach einer weiteren bevorzugten Ausführungsform wird der Schlammspiegel im FAR kurz vor der Wasserentnahme ermittelt und die abzuziehende Wasserfraktion darauf ausgerichtet.

Die Erfindung wird im folgenden anhand einer Zeichnung erläutert. Dabei zeigen die Figuren den Ablauf des Verfahrens.

Die Anlage besteht aus einem Anaerob-Reaktor 1 und einem Faulschlammanreicherungsreaktor (FAR) 2 , die über einen Gasschieber 3 und einen Faulschlammschieber 4 miteinander verbunden sind. Beide Reaktoren 1, 2 sind gasdicht abgesclossen. Der FAR 2 ist mit einer Gaspumpe 5 versehen, die geeignet ist, einen Unterdruck in dem FAR zu bewirken.

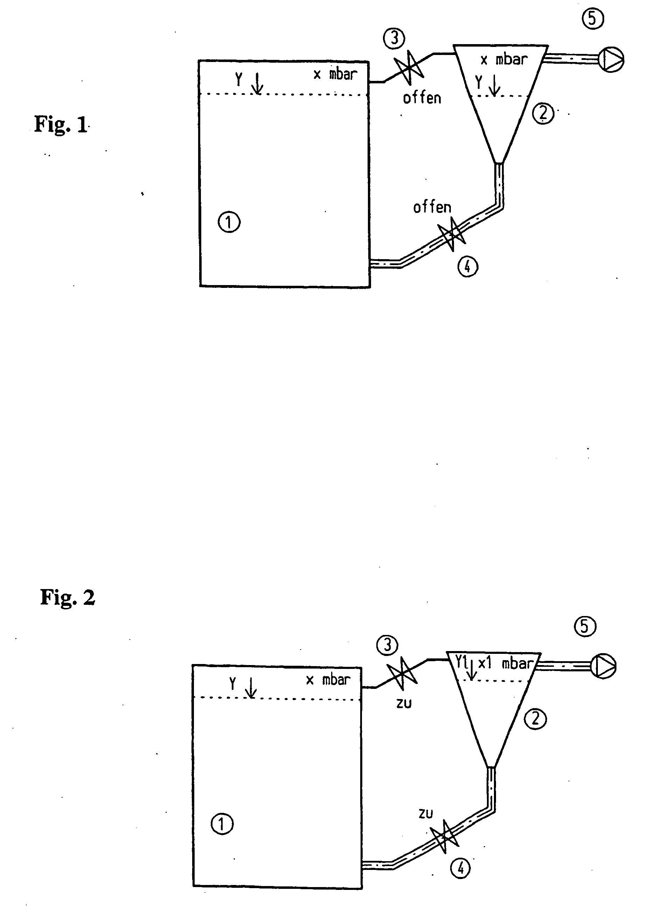

In Fig. 1 sind der Gasschieber 3 und der Faulschlammschieber 4 geöffnet, die Wasserspiegellagen in beiden Behältern (Ruhewasserspiegellagen y) gleichen sich aus; in den Gasphasen beider Reaktoren 1, 2 liegt der gleiche Druck von x mbar vor.

Der sich zyklisch wiederholende Prozess beginnt damit, dass der Gasschieber 3 geschlossen wird und die Gasvakuumpumpe 5 im Gasraum des FAR 2 einen definierten Unterdruck x1 mbar erzeugt. Mit diesem Unterdruck korrespondiert ein neuer höherer Wasserspiegel y1 in dem FAR 2 durch die Zufuhr einer entsprechenden Faulschlammmenge aus dem Reaktor 1. Nachdem x1 mbar erreicht ist, wird der Schieber 4 geschlossen (Fig 2) und der Unterdruck gegebenenfalls auf x2 mbar verstärkt. Danach wird der Unterdruck aufrechterhalten. Die Dauer des Unterdrucks kann je nach Ausfaulungsgrad einige Minuten bis mehrere Stunden andauern. Zweck dieser Unterdruckbehandlung ist, die Gaspartialdrücke (hierbei geht es nahezu ausschließlich um Kohlendioxid CO₂ und Methan CH₄) des Faulschlammes in dem FAR 2 auf unter x mbar zu reduzieren.

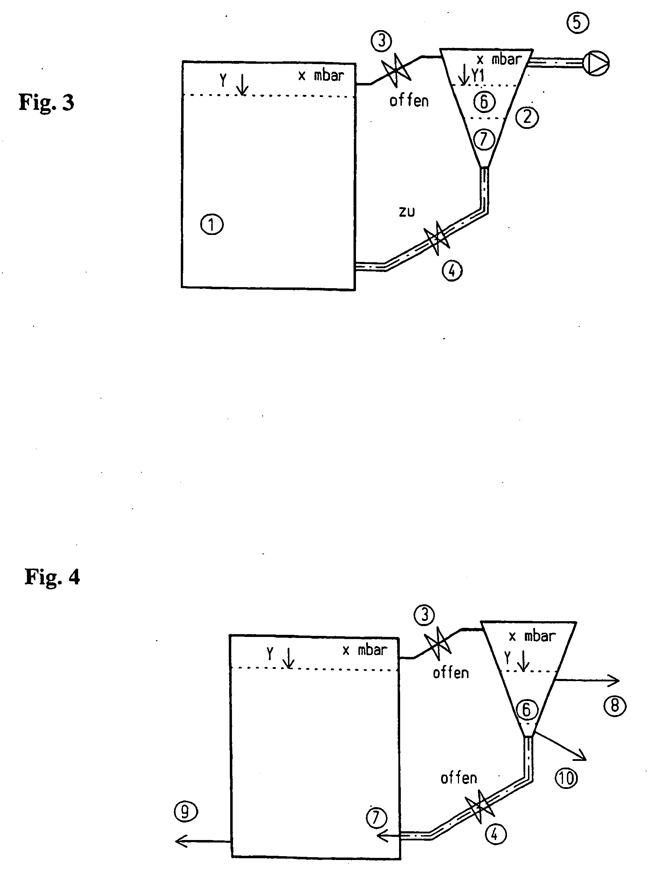

Danach wird der Gasschieber 3 geöffnet, so dass in der Gasphase des FAR 2 wieder ein Gasdruck von ungefähr x mbar entsteht. Da die Gaspartialdrücke in der Flüssigphase des FAR 2 deutlich unter x mbar liegen, wird, bis diese wieder auf Grund der fortlaufenden Faulprozesse auf x mbar ansteigen, ohne störenden Einfluss von aufsteigenden Gasblasen der Faulschlamm in dem FAR 2 sedimentieren und sich eine überstehende nahezu faulschlammfreie rein wässerige Zone 6 bilden (Fig. 3).

Danach wird der Schlammschieber 4 geöffnet. Das Faulschlammkonzentrat 7 im unteren Bereich von des FAR 2 fließt in den Reaktor 1 bis sich beiden Wasserspiegellagen sich wieder auf y einstellen. Aus einem Ablauf 8 wird dann die nahezu faulschlammfreie Wasserfraktion entnommen und das Gesamtsystem kann mit dieser Menge an Rohsubstrat nachgespeist werden (Fig. 4). Danach kann ein neuer Anreicherungszyklus beginnen.

Diese diskontinuierlichen Anreicherungszyklen verlaufen so lange bzw. so oft, bis der maximal möglich bzw. gewünschte TS-Gehalt in dem Reaktor 1 erreicht ist.

Danach kann Netto-Überschussschlammproduktion an Faulschlamm mit den entnommenen Wasserfraktionen entnommen werden. Bevor dieser Zustand entsteht, kann optional über eine weitere Entnahmestelle 9 das Faulschlammkonzentrat entnommen werden.

Die Vorteile dieser Prozessführung lassen sich wie folgt benennen:
I) Es erfolgt eine sehr weitgehende Entkoppelung des partikulären Anteils des Faulschlamms von der Aufenthaltszeit des hydraulischen Anteils des zugeführten Abwassers/Schlammes. Durch diese Prozessführung kann die Aufenthaltszeit des Faulschlammes auf das gewünschte Schlammalter von z.B. 20 Tagen eingestellt werden, obwohl die Aufenthaltszeit des hydraulischen Anteils beispielsweise nur 5 Tage beträgt.
II) Der Aufbau und Betrieb des Faulschlammanreicherungsreaktors (FAR) ist denkbar einfach. In diesem FAR befinden sich keinerlei Aggregate/Geräte oder bewegliche Teile, die eine Wartung erschweren würden. Der Automatikbetrieb beruht ausschließlich auf den externen automatischen Schiebern 3 und 4 und der Gaspumpe 5.
III) Der FAR kann auch (Fig. 4) in einen Stufen- oder Parallelbetrieb integriert werden: Sowohl parallel betriebene Reaktoren als auch eine zweite Stufe können mit einem FAR verbunden und die Faulschlammanreicherung in jede beliebige Richtung geleitet werden.

## Patentansprüche

1. Verfahren zur anaeroben Behandlung von Abwassern und Schlämmen in einer mit einem Faulreaktor (1) und einem mit diesem verbundenen Anreicherungsreaktor (2) versehenen Anlage, bei dem ein Teil des ausfaulenden Schlamms diskontinuierlich abgeführt und in den Faulreaktor (1) rückgeführt wird,
**gekennzeichnet durch**
Schließen eines in einer die beiden Reaktoren (1, 2) verbindenden Gasleitung vorgesehenen Gasschiebers (3) bei geöffnetem in einer die beiden Reaktoren (1, 2) verbindenden Schlammleitung vorgesehenen Schlammschieber (4),
Betätigen einer einen Unterdruck in dem Gasraum des Anreicheruragsreäktors (2) erzeugenden Gaspumpe (5) unter Zuführen von Faulschlamm aus dem Faulreaktor (1) in den Anreicheruagsreaktor (2),
Schließen des Schlammschiebers (4),
Aufrechterhalten des Unterdrucks im Gasraum des Anreicherungsreaktors (2),
Öffnen des Gasschiebers (3),
Öffnen des Schlammschiebers (4) unter Bewirken eines Rückflusses von Faulschlammkonzentrat (7) aus dem Anreicherungsreaktor (2) in den Faulreaktor (1),
Abziehen einer in dem Anreicherungsreaktor (2) überstehenden faulschlammfreien wässrigen Zone (6), und
Wiederholen der vorgenannte Verfahrensschritte.

2. Verfahren nach Anspruch 1, **gekennzeichnet durch** Verstärken des Unterdrucks nach dem Schließen des Schlammschiebers (4).

## Claims

1. Method for the anaerobic treatment of waste water and sludge in a plant provided with a digestion reactor (1) and an enrichment reactor (2) connected thereto, where part of the sludge being digested is removed discontinuously and fed back into the digestion reactor (1),
**characterized by**
closing a gas valve (3) provided in the gas line connecting the two reactors (1, 2) in the case of an open sludge valve (4) provided in a sludge line connecting the two reactors (1,2),
operating a gas pump (5) generating a negative pressure in the gas space of the enrichment reactor (2) while feeding digestion sludge from the digestion reactor (1) into the enrichment reactor (2),
closing the sludge valve (4),
maintaining the negative pressure in the gas space of the enrichment reactor (2),
opening the gas valve (3),
opening the sludge valve (4) while causing a backflow of digestion sludge concentrate (7) from the enrichment reactor (2) into the digestion reactor (1),
withdrawing an aqueous zone (6) free of digestion sludge, projecting in the enrichment reactor (2); and
repeating the process steps mentioned above.

2. Method according to Claim 1, **characterized by** increasing the negative pressure after closing the sludge valve (4)

## Revendications

1. Procédé pour le traitement anaérobie des eaux usées et des boues dans une installation pourvue d'un réacteur de digestion (1) et d'un réacteur d'enrichissement (2) relié à ce dernier, dans lequel une partie de la boue soumise à la digestion est évacuée de manière discontinue et ramenée dans ledit réacteur de digestion (1),
**caractérisé par** le fait
de fermer une vanne à gaz (3) prévue dans une conduite de gaz reliant les deux réacteurs (1, 2) entre eux, lorsque la vanne pour boues (4) prévue dans une conduite à boues reliant les deux réacteurs (1, 2) est ouverte,
d'actionner une pompe à gaz (5) qui génère une dépression dans la chambre à gaz du réacteur d'enrichissement (2), tout en amenant de la boue de digestion en provenance du réacteur de digestion (1) au réacteur d'enrichissement (2),
de fermer ladite vanne pour boues (4),
de maintenir la dépression dans la chambre à gaz du réacteur d'enrichissement (2),
d'ouvrir ladite vanne à gaz (3),
d'ouvrir ladite vanne pour boues (4) en provoquant un reflux de concentré de boues de digestion (7) provenant du réacteur d'enrichissement (2), dans le réacteur de digestion (1),
d'enlever une zone aqueuse exempte de boue de digestion (6) qui surnage dans ledit réacteur d'enrichissement (2), et
de répéter les étapes susmentionnées du procédé.

2. Procédé selon la revendication 1, **caractérisé par** le fait de renforcer la dépression après avoir fermé ladite vanne pour boues (4).
